# EUROPEAN PATENT APPLICATION

(11) **EP 4 144 218 A1**
(43) Date of publication of application: **08.03.2023**
(21) Application number: 21817783.0
(22) Date of filing: 02.06.2021
(51) Int. Cl.: A01N 59/00, A01N 25/04, A01P 1/00, A01P 3/00, A61L 2/18, C02F 1/48

(54) **STERILIZING LIQUID AND METHOD FOR PRODUCING SAME**

(30) Priority: 02.06.2020 JP 2020096108
(71) Applicant: University of Hyogo, Kobe-shi, Hyogo 651-2197 (JP); Kabushiki Kaisha Dainichi Seisakusho, Takasago-shi, Hyogo 676-0815 (JP)
(72) Inventor: OKA Yoshihiro, Kobe-shi, Hyogo 651-2197 (JP); HASHIMOTO Tomohiro, Takasago-shi, Hyogo 676-0815 (JP)
(74) Representative: Cabinet Beau de Loménie
(86) International application number: PCT/JP2021/020982
(87) International publication number: WO 2021/246439

(57) **Abstract**

To provide a sterilizing liquid and a method of producing the same that have no restrictions on application methods and usage and can be widely used for sterilization, a sterilizing liquid including water containing reactive oxygen species and a nanoparticle catalyst in a stationary state is produced by causing cavitation in water having a conductivity of 2000 µS/cm or less and COD of 2000 ppm or less while causing the water to flow by a stirrer 2, and causing generation of plasma by a plasma generation mechanism 3 in which a pulse voltage is applied across electrodes 31 in the water including air bubbles mainly containing water vapor generated by the cavitation.

## Description

### TECHNICAL FIELD

The present invention relates to a sterilizing liquid and a method of producing the same that can be widely used for sterilization in fields of agriculture, food, hygiene, medical treatment, and the like.

### BACKGROUND ART

Conventionally, there has been proposed a sterilizing method which involves causing cavitation in a liquid substance, generating plasma in air bubbles generated by the cavitation to produce reactive oxygen species such as hydroxyl radicals (.OH) in the liquid substance, and making the liquid substance free from microorganisms by oxidizability of the reactive oxygen species (see Patent Documents 1 to 3, for example).

### CITATION LIST

### PATENT LITERATURE

Patent Document 1: Japanese Unexamined Patent Application Publication No. 2011-41914
Patent Document 2: Japanese Unexamined Patent Application Publication No. 2015-3297
Patent Document 3: Japanese Unexamined Patent Application Publication No. 2017-176201

### SUMMARY OF INVENTION

### TECHNICAL PROBLEM

The method of sterilizing a liquid substance described in Patent Documents 1 to 3 can obtain a strong sterilizing power because this method makes the liquid substance free from microorganisms by oxidizability of reactive oxygen species. However, this method has a problem that application methods and usage are restricted because it is necessary to put the liquid substance to be sterilized directly into a sterilization device.

In view of the problem of the above-described conventional method of sterilizing a liquid substance, it is an object of the present invention to provide a sterilizing liquid and a method of producing the same that have no restrictions on application methods and usage and can be widely used for sterilization.

### SOLUTION TO PROBLEM

In order to achieve the above object, a sterilizing liquid of the present invention is characterized by including water containing reactive oxygen species and a nanoparticle catalyst (including secondary particles formed by aggregated nanoparticles) in a stationary state.

In this case, the sterilizing liquid is characterized in that a concentration of hydrogen peroxide in the reactive oxygen species immediately after production of the sterilizing liquid is 30 to 2000 ppm, and a concentration of the nanoparticle catalyst is 16 ppm or more.

Here, "immediately after production of the sterilizing liquid" means within a few minutes after production of the sterilizing liquid.

Further, the sterilizing liquid is characterized in that a sterilizing effect lasts for more than a few hours.

A method of producing a sterilizing liquid of the present invention for producing the above-described sterilizing liquid is characterized by causing cavitation in water having a conductivity of 2000 µS/cm or less and COD of 2000 ppm or less while causing the water to flow, and generating plasma by a plasma generation mechanism in which a pulse voltage is applied across electrodes in the water including air bubbles mainly containing water vapor generated by the cavitation, thereby producing a sterilizing liquid including water containing reactive oxygen species and a nanoparticle catalyst in a stationary state.

### ADVANTAGEOUS EFFECT OF INVENTION

According to the sterilizing liquid and the method of producing the same of the present invention, it is not necessary to put a liquid substance to be sterilized directly into a sterilization device, unlike the liquid substance sterilizing method described in Patent Documents 1 to 3. In particular, the sterilizing liquid of the present invention is secure and safe because it uses water as its raw material, and the reactive oxygen species that are components exerting a sterilizing action finally turn back to water. Therefore, the present invention can provide the sterilizing liquid and the method of producing the same that have no restrictions on application methods and usage and can be widely used for sterilization.

### BRIEF DESCRIPTION OF DRAWINGS

FIG. 1 is a conceptual diagram showing an example of a sterilizing liquid producing device to which a method of producing a sterilizing liquid according to the present invention is applied.
FIG. 2 shows the principle of decomposition of organic matter by reactive oxygen species, and an oxidation potential and a binding energy for various materials including the reactive oxygen species.
FIG. 3 shows a relation between processing time, a pH value, a conductivity, a hydrogen peroxide concentration, and the amount of electrode wear for each electrode material.
FIG. 4 shows a relation between time elapsed after CBP processing and a survival rate of bacteria.
FIG. 5 shows change of an absorption peak value of an MB solution with time (a relation between elapsed time and the absorption peak value).
FIG. 6 shows an emission spectrum of CBP using emission spectrochemical analysis (a relation between a wavelength and an emission intensity).
FIG. 7 shows dependency of an emission peak value of hydroxyl radicals (·OH) (309.6 nm) on CBP processing time (a relation between the processing time and an emission intensity).
FIG. 8 shows change of an absorption peak value (664 nm) of an MB solution with time (a relation between elapsed time and the absorption peak value).
FIG. 9 shows a reaction speed constant k obtained from the slope in FIG. 8 (a relation between elapsed time and the reaction speed constant).
FIG. 10 shows dependency of a hydrogen peroxide concentration on CBP processing time (a relation between the processing time and the hydrogen peroxide concentration).
FIG. 11 shows dependency of the amount of electrode wear on CBP processing time (a relation between the processing time and the amount of electrode wear).
FIG. 12 shows an ultraviolet-visible absorption spectrum of a methylene blue solution processed under a processing condition for a sterilizing liquid producing device (tₚ = 0.5 min) (a relation between a wavelength and an absorptance for each elapsed time).
FIG. 13 shows an ultraviolet-visible absorption spectrum of a methylene blue solution processed under a processing condition for a sterilizing liquid producing device (tₚ = 1 min) (a relation between a wavelength and an absorptance for each elapsed time).
FIG. 14 shows an ultraviolet-visible absorption spectrum of a methylene blue solution processed under a processing condition for a sterilizing liquid producing device (tₚ = 2 min) (a relation between a wavelength and an absorptance for each elapsed time).
FIG. 15 is an explanatory diagram of an experimental procedure of a sterilizing effect test (2).
FIG. 16 shows a relation between elapsed time after production of a sterilizing liquid and an MB decomposition rate for each processing condition for a sterilizing liquid producing device.
FIG. 17 is an explanatory diagram of a factor of a sterilizing effect provided by a sterilizing liquid.
FIG. 18 is an explanatory diagram of a factor of a sterilizing effect provided by a sterilizing liquid.
FIG. 19 is an explanatory diagram of a factor of a sterilizing effect provided by a sterilizing liquid.
FIG. 20 is an explanatory diagram of a factor of a sterilizing effect provided by a sterilizing liquid.
FIG. 21 shows a relation between elapsed time and an MB decomposition rate for a sterilizing liquid and hydrogen peroxide.
FIG. 22 shows a relation between processing conditions for a sterilizing liquid producing device (tₚ = 1 min to 30 min) and the amount of wear of a tungsten (W) electrode.
FIG. 23 shows a relation between elapsed time after production of a sterilizing liquid and a pH value for each processing condition for a sterilizing liquid producing device (tₚ = 1 min to 30 min).
FIG. 24 shows a relation between elapsed time after production of a sterilizing liquid and a conductivity for each processing condition for a sterilizing liquid producing device (tₚ = 1 min to 30 min).
FIG. 25 shows a relation between elapsed time after production of a sterilizing liquid and an ORP for each processing condition for a sterilizing liquid producing device (tₚ = 1 min to 30 min).
FIG. 26 shows a relation between elapsed time after production of a sterilizing liquid and a hydrogen peroxide concentration for each processing condition for a sterilizing liquid producing device (tₚ = 1 min to 30 min).
FIG. 27 shows a relation between CBP processing time and a generation rate of in-liquid plasma.
FIG. 28 shows a relation between elapsed time after production of a sterilizing liquid and an MB decomposition rate for each processing condition (each electrode material) for a sterilizing liquid producing device.
FIG. 29 shows a relation between elapsed time after production of a sterilizing liquid and an MB decomposition rate for each processing condition (each electrode material) for a sterilizing liquid producing device.
FIG. 30(a) shows a relation between an electrode material and a pH value for each processing condition (each electrode material) of a sterilizing liquid producing device, and FIG. 30(b) shows a relation between that electrode material and a conductivity.
FIG. 31(a) shows a relation between an electrode material and an ORP for each processing condition (each electrode material) of a sterilizing liquid producing device, and FIG. 31(b) shows a relation between that electrode material and a hydrogen peroxide concentration.

### DESCRIPTION OF EMBODIMENTS

An embodiment of a sterilizing liquid and a method of producing the sterilizing liquid according to the present invention are described below.

The sterilizing liquid of the present invention includes water containing reactive oxygen species and a nanoparticle catalyst in a stationary state. This sterilizing liquid can be produced by causing cavitation in water having a conductivity of 2000 µS/cm or less and COD of 2000 ppm or less while causing the water to flow, and generating plasma by a plasma generation mechanism in which a pulse voltage is applied across electrodes in the water including air bubbles mainly containing water vapor generated by the cavitation.

A device of producing the sterilizing liquid can be a conventionally known device, that is, a mechanism of causing cavitation in water and generating plasma using a plasma generation mechanism in which a pulse voltage is applied across electrodes in the water including air bubbles mainly containing water vapor generated by the cavitation. More specifically, such a device of producing the sterilizing liquid can be a device obtained by combining a cavitation generation mechanism for causing cavitation in water by changing the flow-passage cross-sectional area with a nozzle, an obstacle, etc., and the plasma generation mechanism with each other as described in Patent Documents 1 and 2, a device obtained by combining a cavitation generation mechanism for causing cavitation in water by rotating a rotor and the plasma generation mechanism with each other as described in Patent Document 3, or the like.

Here, an example is described which uses the device obtained by combining the cavitation generation mechanism that causes cavitation in water by rotating the rotor and the plasma generation mechanism with each other as the sterilizing liquid producing device.

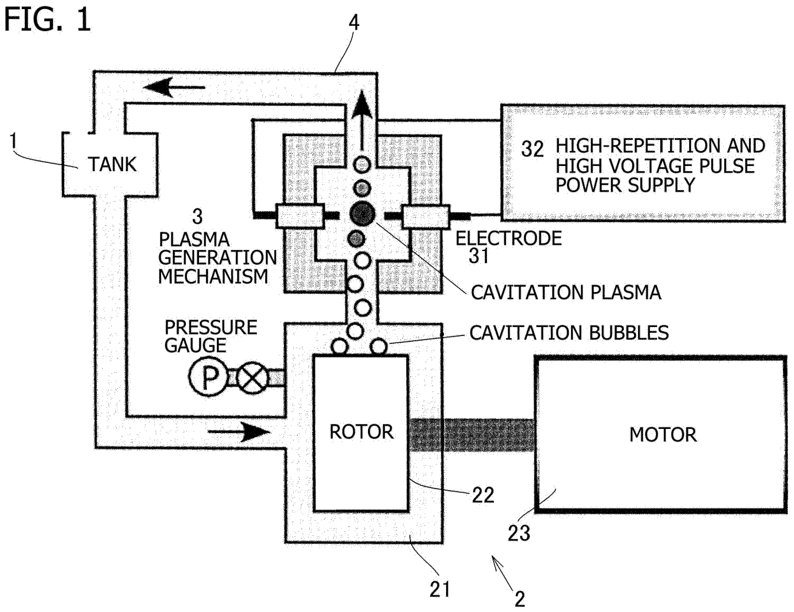

This sterilizing liquid producing device is configured to include a tank 1 that stores water therein, a stirrer 2 as a cavitation generation mechanism that stirs the water supplied from the tank 1, a plasma generation mechanism 3 that causes cavitation by the stirrer 2 and causes generation of plasma in the water including air bubbles (cavitation bubbles) mainly containing water vapor generated by the cavitation, and a conduit line 4 that connects these mechanisms and causes the water to circulate, as shown in FIG. 1.

Causing the water to circulate is not essential. A configuration for one-path processing may be used, as long as processing efficiency is increased more, for example, by providing a plurality of plasma generation mechanisms and/or installing a plurality of pairs of electrodes.

The stirrer 2 is configured to include a rotor 22 that is provided in a casing 21 to be rotatable and is concentric with the casing 21, a motor 23 that drives the rotor 22 to rotate, and the like.

The plasma generation mechanism 3 is configured to include electrodes 31 formed by a conductor, a pulse power supply 32 that applies, for example, a voltage equal to or higher than a discharge inception voltage and a pulse voltage with a pulse width of 1.5 µs or less and a repetition frequency of 100 kHz or more across the electrodes 31, and the like. By the plasma generation mechanism 3, vapor is ionized (changed to plasma) through high voltage breakdown discharge caused by the pulse voltage in an insulating air-bubble region, whereby in-liquid plasma (cavitation plasma, which may be referred to as "CBP (Cavitation bubble plasma)" in the present specification) is generated.

The form of discharge caused by the pulse voltage is preferably glow discharge (low-temperature plasma produced by glow discharge). The glow discharge can synthesize a nanoparticle catalyst in the crystal form, the quasicrystal form, the amorphous form, and the like at low temperature in an energy-efficient manner; the nanoparticle catalyst is made of, for example, an inorganic compound such as a metal or metal oxide arising from a wear component of the electrode 31, or a sulfide or chloride arising from impurities contained in the water produced by the in-liquid plasma. That is, the nanoparticle catalyst is nanoparticles of a component of the electrode 31. In the case where the electrodes are made of a metal, the nanoparticles are metal nanoparticles at the moment when being generated. Depending on the type of the metal, the nanoparticles are oxidized or, when chlorine or sulfur is contained in the water, chlorinated or sulfurized (the nanoparticles may be turned into a compound with a substance contained in impurities).

The flow rate of water near the electrodes 31 in the plasma generation mechanism 3 is about 10 m/s and desirably 5 m/s or more.

The electrodes 31 are preferably disposed to be opposed to each other in the direction perpendicular to the flow of water. However, other arrangements including the inverted V-shape arrangement can be employed, as long as plasma can be produced.

As the material for the electrodes 31, any of conductor materials including: metals such as tungsten, copper, iron, silver, gold, platinum, aluminum, scandium, titanium, vanadium, chromium, manganese, cobalt, nickel, zinc, gallium, germanium, yttrium, zirconium, molybdenum, technetium, ruthenium, rhodium, palladium, cadmium, indium, tin, antimony, lanthanoid, hafnium, tantalum, rhenium, osmium, iridium, thallium, bismuth, and polonium; carbon; conductive diamond; alloys of these materials; composite materials of these materials (including a member covered with a thin film formed by plating, dry coating, or the like); and oxides (including a resultant material obtained by a reaction of the surface of the electrodes 31 with water) can be selected depending on the usage. The electrodes 31 disposed to be opposed to each other can differ in the material to be used and/or the size. For example, the electrodes 31 are made of gold and silver, respectively.

The electrodes 31 may have a shape of a cylinder, a prism, an elliptical cylinder, a cone, or a pyramid. Although one pair of electrodes 31 is enough, two or more pairs of electrodes 31 may be provided in order to increase processing efficiency more. Regarding the plasma generation mechanism, one set is enough. However, two or more sets may be provided in order to increase the processing efficiency more.

One of the electrodes 31 may be grounded or not grounded. The configuration in which one of the electrodes 31 is not grounded is safer because a discharge path is limited between the electrodes.

Further, water can be processed at a temperature of 50°C or less by operating a cooler provided in the sterilizing liquid producing device, for example, a jacket cooling unit (not shown) provided in the stirrer 2, as necessary.

In the water thus produced by in-liquid plasma, hydrogen peroxide or the like as reactive oxygen species is stably present, in addition to the nanoparticle catalyst, even in a stationary state in which the operation of the sterilizing liquid producing device is stopped. Therefore, this water can be used as a sterilizing liquid having a sterilizing action due to the oxidizability of hydrogen peroxide.

This sterilizing liquid continuously produces reactive oxygen species, for example, hydroxyl radicals (·OH) that have the highest oxidizability among the reactive oxygen species due to a sterilizing action by long-lived reactive oxygen species (superoxide anion radicals (·O₂⁻), hydroperoxy radicals (HOO·), and hydrogen peroxide (H₂O₂)) present in the sterilizing liquid and a catalyst action of the nanoparticle catalyst present in the sterilizing liquid in which hydrogen peroxide (H₂O₂) is present, whereby the sterilizing effect is further enhanced, and the effect lasts a long time.

Therefore, regarding the sterilizing liquid, the amounts of hydrogen peroxide and the nanoparticle catalyst that are present in the sterilizing liquid are important. According to the present invention, a large amount of sterilizing liquid can be produced only from water as a raw material in a simple, fast, and simultaneous manner.

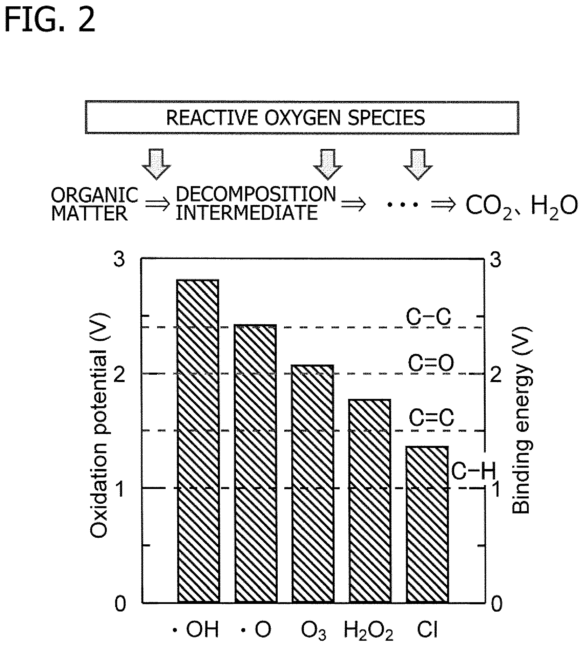

FIG. 2 shows the principle of decomposing (killing) organic matter (including microorganisms (e.g., viruses, bacteria, fungi, and protozoa) by reactive oxygen species, and an oxidation potential and a binding energy for various materials including the reactive oxygen species.

As is apparent from FIG. 2, the reactive oxygen species such as hydroxyl radicals (·OH) have a strong action of decomposing (killing) organic matter (including microorganisms (e.g., viruses, bacteria, fungi, and protozoa), and therefore can be used as a sterilizing liquid.

### EXAMPLES

Next, a test is described which was conducted using a sterilizing liquid producing device (specifically, a "cavitation plasma device" manufactured by Nihon Spindle Manufacturing Co., Ltd.).

### [Processing condition for sterilizing liquid producing device]

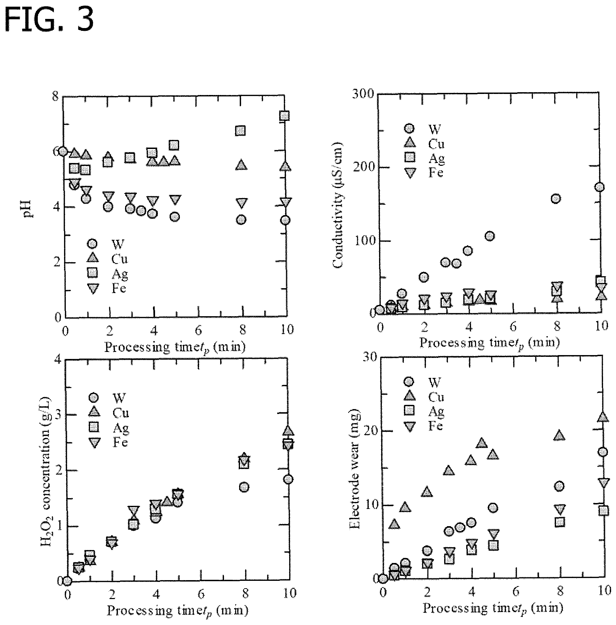

A processing condition (preferable ranges) for a sterilizing liquid producing device is shown in Table 1, and relations between processing time and a pH value, a conductivity, a hydrogen peroxide concentration, and the amount of electrode wear for each electrode material are shown in FIG. 3.

**[Table 1]**

| Preferable Ranges of CBP Processing Condition | | |
|---|---|---|
| Solution | Type | Ion-Exchanged Water |
| | Weight | 250 g |
| | Initial pH (preferably neutral) | 5 to 9 |
| | Initial Conductivity (low conductivity is preferable) | 0.01 to 2000 µS/cm |
| | Initial Water Temperature | 15 °C |
| | Initial COD (low COD is preferable) | 0.01 to 2000 ppm |
| Stirrer | Number of Revolutions (preferably high) | 2400 to 7200 rpm |
| Power Supply | Applied Voltage (preferably about 10 kV) | ± 1 to 20 kV |
| | Pulse Width (preferably about 1.0 µs) | 0.5 to 2.0 µs |
| | Repetition Frequency (high frequency is preferable) | 0.1 to 500 kHz |
| Electrode | Material (conductor stable in water) | W, Ag, Cu, Fe, alloys, and the like |
| | Diameter (preferably about 2 mm) | 0.2 to 10 mm |
| | Gap Length (preferably 1 to 2 mm) | 0.2 to 10 mm |
| Processing Time tₚ (preferably 5 min) | | 0.1 to 60 min |

As for Table 1 and FIG. 3, the following can be said.
- As an initial conductivity is lower, a plasma production rate (a ratio of the number of pulses produced by plasma to the number of applied pulses) is higher, and therefore reactive oxygen sterilizing water can be efficiently produced. Here, "initial means "before plasma processing". This is the same as to other items.
- Higher initial COD is not preferable because produced reactive oxygen species are consumed.
- Other contaminants have no effect as long as the initial conductivity, an initial PH value, and the initial COD are within preferable ranges, respectively. Therefore, it is preferable to use purified water such as ion-exchanged water as water that is a raw material of processing. However, the raw material is not limited thereto.
- As the number of revolutions of a stirrer becomes larger, cavitation bubbles increase. Therefore, the plasma production rate becomes high, and reactive oxygen sterilizing water can be efficiently produced.
- When an applied voltage is excessively low, plasma is not lit up. When the applied voltage is high, the plasma production rate becomes high. However, an excessively high applied voltage is not preferable because transition from glow discharge, which is preferable, to arc discharge occurs.
- Plasma is not lit up when the pulse width is excessively short, whereas the plasma production rate becomes high when the pulse width is long. However, an excessively long pulse width is not preferable because transition to arc discharge occurs.
- As a repetition frequency is higher, the plasma production rate is higher, and plasma can be produced more stably.
- A pulse voltage may be bipolar or have a positive polarity or a negative polarity.
- It suffices that the electrode material is any conductor, as long as it is stable in water. The electrode material may be a metal, an alloy, or carbon.
- Since a nanoparticle catalyst such as an electrode component is mixed as impurities in the reactive oxygen sterilizing water, the electrode material needs to be selected depending on usage.
- When the electrode diameter is excessively thin, concentration of an electric field occurs, and plasma can be lit up easily. However, transition to arc discharge can occur easily. When the electrode diameter is excessively thick, plasma is less likely to be lit up.
- When the gap length between the electrodes is excessively short, transition to arc discharge can easily occur. When this gap length is excessively long, plasma is less likely to be lit up.
- When the processing time is excessively short, the produced amounts of reactive oxygen species and nanoparticle catalyst are reduced. When the processing time is excessively long, the reactive oxygen species and the nanoparticle catalyst that are produced make the conductivity higher and the plasma generation rate lower. The processing time is normally about 2 to 10 minutes, preferably about 3 to 8 minutes, and more preferably about 5 minutes. Here, the sterilizing liquid producing device is set in such a manner that, when the number of revolutions of the stirrer is 7200 rpm, 250 mL of water circulates in the device once per second.

### [Residual sterilizing effect test]

Next, a residual sterilizing effect test was conducted using Escherichia coli as a model microorganism in accordance with the following test method.

### (1) Test bacteria

Escherichia coli NBRC 3301

### (2) Medium and culture condition for measurement of number of bacteria

SCDLP agar medium (manufactured by Nihon Pharmaceutical Co., Ltd.), pour plate cultural method, 35°C ± 1°C, for 2 days

### (3) Preparation of test bacteria solution

The test bacteria were cultured on a nutrient agar medium (manufactured by Eiken Chemical Co., Ltd.) at 35°C ± 1°C for 18 to 24 hours, then suspended in purified water, and adjusted to set the number of bacteria to about 107 mL. A test bacteria solution was thus prepared.

### (4) Pre-processing for sterilizing liquid producing device

A sodium hypochlorite solution prepared to have a concentration of about 0.02% was put into the sterilizing liquid producing device, and the device was operated for 15 minutes under a condition without plasma processing. After drainage, the inside of the sterilizing liquid producing device was rinsed with tap water, tap water containing 0.002% sodium thiosulfate, and distilled water in this order, followed by drainage.

### (5) Test operation

Into the sterilizing liquid producing device after pre-processing, 250 mL of purified water (ion-exchanged water) was put. The device was then operated for 5 minutes under a processing condition shown in Table 2. Sample water was thus obtained. The sample water was collected in a sterile container made of synthetic resin and left to stand for a predetermined time. Then, 0.1mL of the test bacteria solution was inoculated into 10mL of the sample water after standing for the predetermined time, to obtain a test solution. Thereafter, the test solution was diluted 100 times with an SCDLP medium (manufactured by Nihon Pharmaceutical Co., Ltd.), and the number of viable bacteria in the test solution was measured using a medium for measuring the number of bacteria.

As a control, purified water (ion-exchanged water) with the test bacteria solution inoculated thereto was also tested in the same manner, and the number of viable bacteria in the test solution was measured using the medium for measuring the number of bacteria.

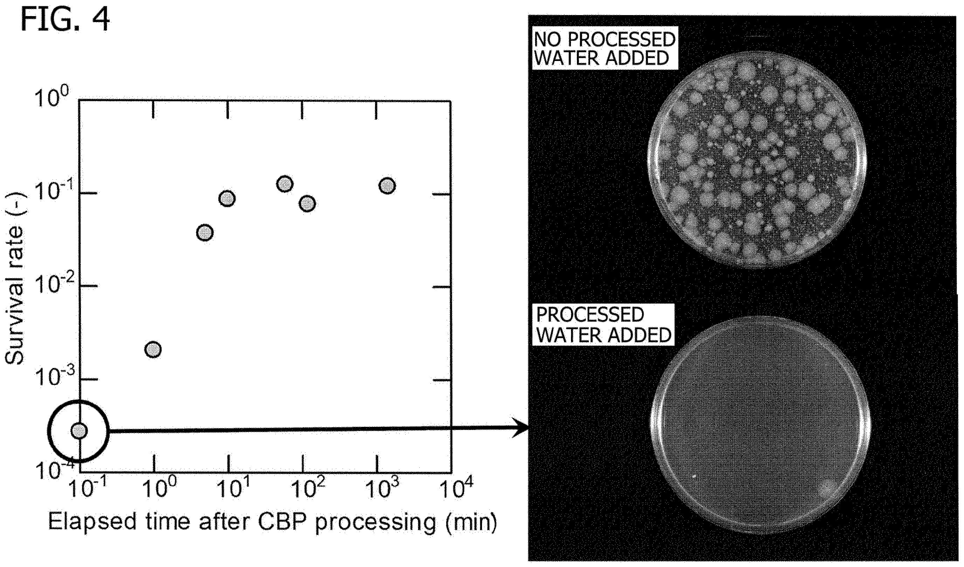

FIG. 4 shows each residual sterilizing effect test (a relation between elapsed time after CBP processing and a survival rate of bacteria).

**[Table 2]**

| CBP Processing Condition | | |
|---|---|---|
| Solution | Type | Ion-Exchanged Water |
| | Weight | 250 g |
| | Initial pH | 6 |
| | Initial Conductivity | 2 µS/cm |
| | Initial Water temperature | 15 °C |
| | Initial COD | 2 ppm |
| Stirrer | Number of Revolutions | 7200 rpm |
| Power Supply | Applied Voltage | ± 4 kV |
| | Pulse Width | 0.8 µs |
| | Repetition Frequency | 200 kHz |
| Electrode | Material | W |
| | Diameter | 2.0 mm |
| | Gap Length | 1.0 mm |
| Processing Time *tₚ* | | 5 min |

| | | |
|---|---|---|
| ^{∗} Cooling with cooling water is performed. A solution temperature at the time of processing is about 40°C. | | |

The residual sterilizing effect test shown in FIG. 4 revealed the following.
- After the CBP processing (after production of the sterilizing liquid), the survival rate of bacteria increases and the sterilizing effect decreases, as time elapses.
- Within a few minutes after the CBP processing (after production of the sterilizing liquid), the survival rate of bacteria is low, and a sufficient sterilizing effect is obtained. Particularly, within 6 seconds, the survival rate of bacteria is 0.1% or less, and a remarkable sterilizing effect is obtained.

### [Sterilizing effect test (1)]

Next, a sterilizing effect test (1) was conducted using an aqueous solution of organic matter (methylene blue (which may be referred to as "MB" in the present specification) in place of microorganisms in the following procedure.

The reason why the aqueous solution of organic matter (methylene blue) was used in place of microorganisms is that a factor of a sterilizing effect and a factor of an effect of decomposing of the organic matter (methylene blue) are both reactive oxygen species, and therefore methylene blue, which can be evaluated more easily, is used (the same applies to a sterilizing effect test (2) described later).

Tables 3 and 4 show processing conditions for a sterilizing liquid producing device.

**[Table 3]**

| CBP Processing Condition | | |
|---|---|---|
| Solution | Type | MB solution |
| | Weight | 260 g |
| | MB concentration | 8.6 ppm |
| | Initial pH | 6 |
| | Initial Conductivity | 4 to 5 µS/cm |
| | Initial Water Temperature | 30°C |
| Power Supply | Voltage Peak Value | 10 kV |
| | Pulse Width | 1.0 µs |
| | Repetition Frequency *f* | 200 kHz |
| | Polarity | Bipolar |
| Stirrer | Number of Revolutions | 7200 rpm |
| Electrode | Material | W |
| | Diameter | 2.0 mm |
| | Gap Length | 1.0 mm |
| CBP Processing Time *tₚ* | | 0 to 10 min |
| Elapsed time after processing *tₑ* | | 1 min |

**[Table 4]**

| CBP Processing Condition | | |
|---|---|---|
| Solution | Type | Ion-Exchanged Water |
| | Weight | 260 g |
| | Initial pH | 6 |
| | Initial Conductivity | 1 µS/cm |
| | Initial Water Temperature | 30°C |
| Power Supply | Voltage Peak Value | 10 kV |
| | Pulse Width | 1.0 µs |
| | Repetition Frequency *f* | 200 kHz |
| | Polarity | Bipolar |
| Stirrer | Number of Revolutions | 7200 rpm |
| Electrode | Material | W |
| | Diameter | 2.0 mm |
| | Gap Length | 1.0 mm |
| CBP Processing Time *tₚ* | | 5 (ref.), 0.1 to 20 min |
| Elapsed Time After Processing *tₑ* | | 2 to 30 min |

First, a test was conducted in order to confirm that an organic matter decomposition effect may be greatly affected by residues such as organic matter in the sterilizing liquid producing device.

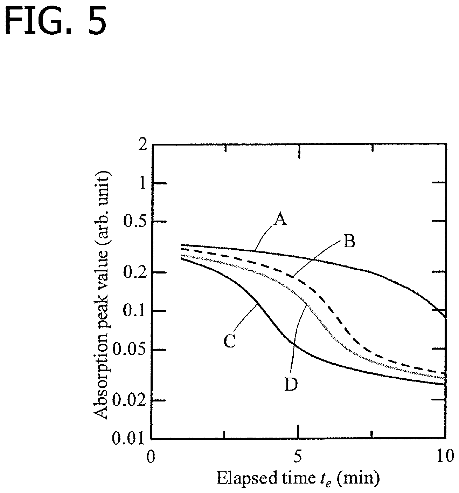

FIG. 5 shows change of an absorption peak value of an MB solution processed under the condition of Table 3 (tₚ = 5 min, tₑ = 0 to 10 min) with time (a relation between elapsed time and the absorption peak value). Here, explanatory notes [A, B, C, and D] respectively represent that pre-processing was performed under conditions of [Table 3 (tₚ = 0 min), Table 3 (tₚ = 5 min), Table 4 (tₚ = 5 min), and Table 4 (tₚ = 10 min)] immediately before the processing.

The change with time was greatly different depending on the pre-processing immediately before the processing. In particular, an MB decomposition speed was greatly reduced after the MB solution was put before the processing. Thus, it was confirmed that the organic matter decomposition effect was greatly affected by residues (contamination) such as organic matter in the sterilizing liquid producing device.

Further, based on the above findings, the inside of the CBP device was washed five times with ion-exchanged water before the test, and glass cells, glass beakers, and a processed liquid discharge port of the CBP device were subjected to ultrasonic cleaning with ion-exchanged water for 5 minutes, whereby contaminants were removed.

In addition, a container (a quartz glass cell (for an ultraviolet-visible absorption spectrum) or a beaker (container)) from which a sterilizing liquid is taken out is also cleaned. It is desirable that a container for storing the sterilizing liquid therein be non-organic and uncontaminated.

Therefore, in the following test, Table 4 (tₚ = 5 min) was set as a standard condition, and a method including the above-described pre-processing was adopted.

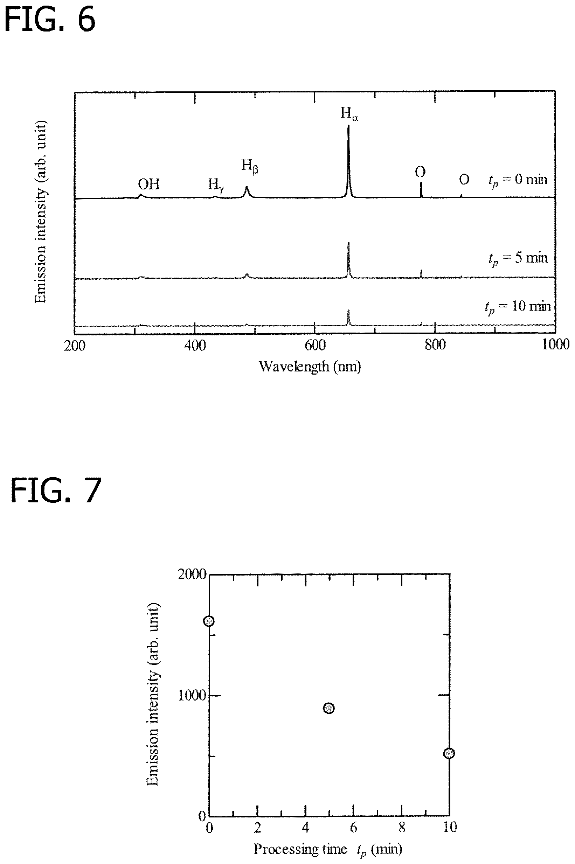

FIG. 6 shows an emission spectrum of CBP using emission spectrochemical analysis (a relation between a wavelength and an emission intensity) in the case of processing under the processing condition for a sterilizing liquid producing device shown in Table 4, and FIG. 7 shows dependency of an emission peak value of hydroxyl radicals (·OH) (309.6 nm) on CBP processing time (a relation between the processing time and an emission intensity) in that case.

These show that OH radicals and O radicals are produced in plasma-processed water processed under the processing condition for the sterilizing liquid producing device.

Next, an organic matter decomposition effect test was conducted in the following procedure.

Immediately after processing under the processing condition for a sterilizing liquid producing device shown in Table 4, 260 µL of an MB solution having a concentration of 8600 ppm was put into the device. The change with time of a peak intensity at a wavelength of 664 nm in an ultraviolet-visible absorption spectrum of an MB solution after the MB solution was put was measured using an ultraviolet-visible spectrophotometer (V-730BIO, JASCO Corporation).

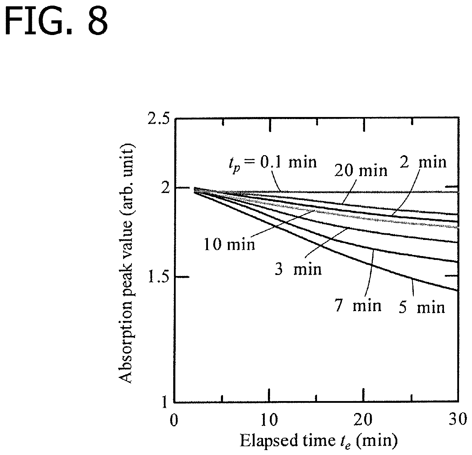

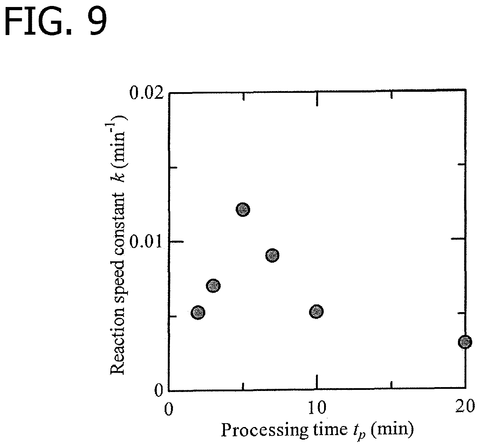

FIG. 8 shows change of an absorption peak value (664 nm) of an MB solution with time (a relation between elapsed time and the absorption peak value), and FIG. 9 shows a reaction speed constant k obtained from the slope in FIG. 8 (a relation between the elapsed time and the reaction speed constant).

As shown in FIG. 8, when tₚ = 0.1 min, the absorption peak value hardly decreases as the elapsed time increases.

Meanwhile, when tₚ = 2 min, the absorption peak value decreases exponentially in a range of tₑ = 2 to 20 min with increase in the elapsed time. After tₑ = 20 min, the slope becomes gentle. At tₑ = 30 min, the absorption peak value decreases to 1.8. The decreased speed is the maximum at tₚ = 5 min, and decreases to 1.4 when the absorption peak value tₑ is 30 min. After tₚ = 5 min, the decrease speed becomes smaller, and when tₚ = 20 min, the absorption peak value decreases only up to 1.82. From these results, it can be said that the decomposition effect remains more in the plasma-processed water in the case of tₚ = 5 min.

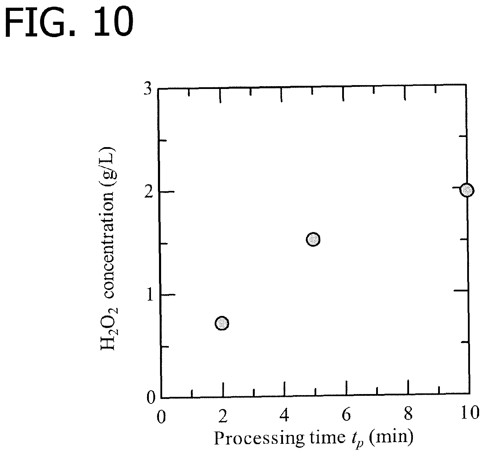

As shown in FIG. 9, in a range of tₚ = 2 to 5 min, the reaction speed constant k linearly increases with increase in the CBP processing time, and k = 0.012 min⁻¹ when tₚ = 5 min. In a range of tₚ = 5 to 10 min, the reaction speed constant k decreases linearly with increase in the CBP processing time, and k = 0.0052 min⁻¹ when tₚ = 10 min. Thereafter, the decrease speed becomes gentle, and k = 0.0030 min⁻¹ when tₚ = 20 min. These results show that the CBP processing time providing the strongest residual decomposition effect is around tₚ = 5 min (a concentration of hydrogen peroxide among reactive oxygen species = 1500 ppm (see FIG. 10)), and the CBP processing time providing the residual decomposition effect is about 2 to 10 minutes (the concentration of hydrogen peroxide among reactive oxygen species = 700 to 2000 ppm (see FIG. 10)) and is preferably about 3 to 8 minutes (the concentration of hydrogen peroxide among reactive oxygen species = 1000 to 1800 ppm (see FIG. 10)).

Here, the hydrogen peroxide concentration is a measurement value immediately after production of a sterilizing liquid (within a few minutes after production of the sterilizing liquid).

FIG. 10 shows dependency of a hydrogen peroxide concentration on CBP processing time (a relation between the processing time and the hydrogen peroxide concentration).

As shown in FIG. 10, as the CBP processing time increases, the hydrogen peroxide concentration increases in a manner close to a saturation tendency. The hydrogen peroxide concentration at tₚ = 10 min is 2.0 g/L (2000 ppm). The cause of production of hydrogen peroxide by CBP processing is considered to be that hydroxyl radicals (·OH) produced by CBP processing are bonded to each other to produce hydrogen peroxide.

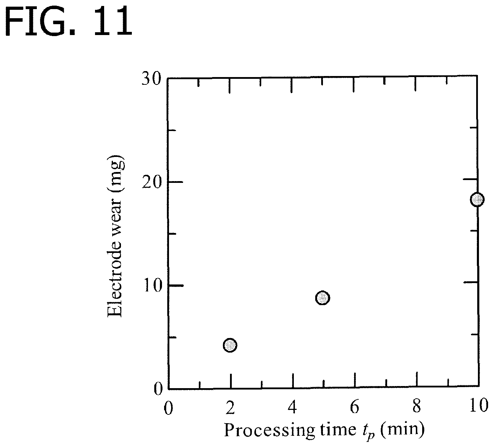

FIG. 11 shows dependency of the amount of electrode wear on CBP processing time (a relation between the processing time and the amount of electrode wear).

As shown in FIG. 11, as the CBP processing time increases, the amount of electrode wear increases linearly. When tₚ = 2 min, the amount of electrode wear becomes 4 mg (the concentration of nanoparticle catalyst = 16 ppm). When tₚ = 10 min, the amount of electrode wear becomes 18 mg (the concentration of nanoparticle catalyst = 72 ppm). These results show that the concentration of nanoparticle catalyst contained in a sterilizing liquid is equal to or higher than 16 ppm (to 72 ppm). The reason why an electrode is worn as the CBP processing time increases is that the electrode becomes nanoparticles due to sputtering by CBP processing.

### [Sterilizing effect test assuming contaminated water]

Next, a sterilizing effect test assuming contaminated water was conducted using an MB solution in place of ion-exchanged water.

This test assumes a case where water to be used is contaminated before CBP processing (a case where COD is high), uses methylene blue in place of contamination, and uses an MB solution obtained by dissolving methylene blue in water before the CBP processing. Here, a test in which plasma processing time is shortened is a test assuming that contamination remains even after the CBP processing.

Table 5 shows a processing condition for a sterilizing liquid producing device.

**[Table 5]**

| CBP Processing Condition | | |
|---|---|---|
| Solution | Type | MB Solution |
| | Weight | 250 g |
| | Initial pH | 6 |
| | Initial Conductivity | 6 µS/cm or less |
| | Initial Water Temperature | 15 °C |
| Stirrer | Number of Revolutions | 7200 rpm |
| Power Supply | Applied Voltage | ± 12 kV |
| | Pulse Width | 0.9 µs |
| | Repetition Frequency | 200 kHz |
| Electrode | Material | Cu |
| | Diameter | 2.0 mm |
| | Gap Length | 1.0 mm |
| Processing Time *tₚ* | | 0.5, 1, and 2 min |

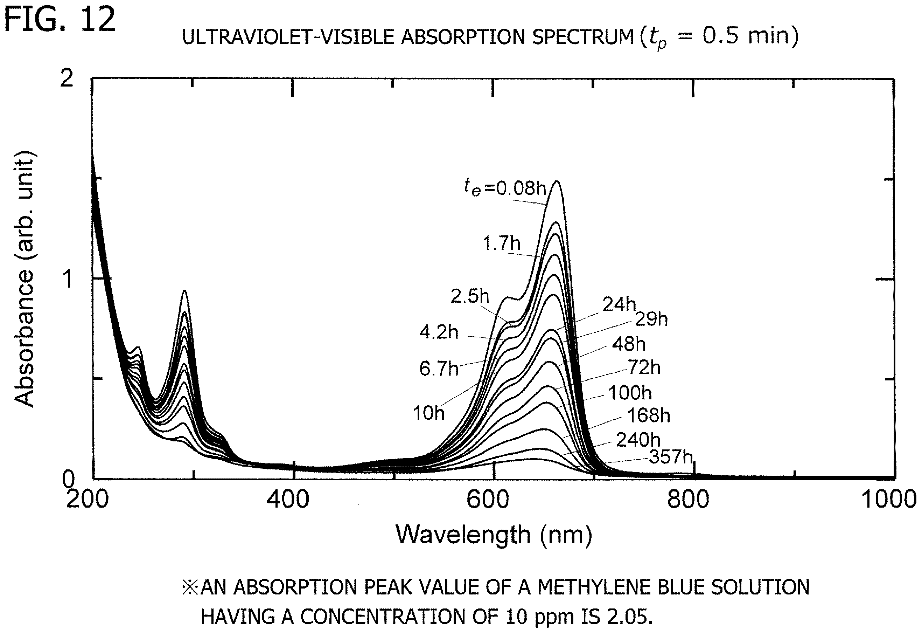

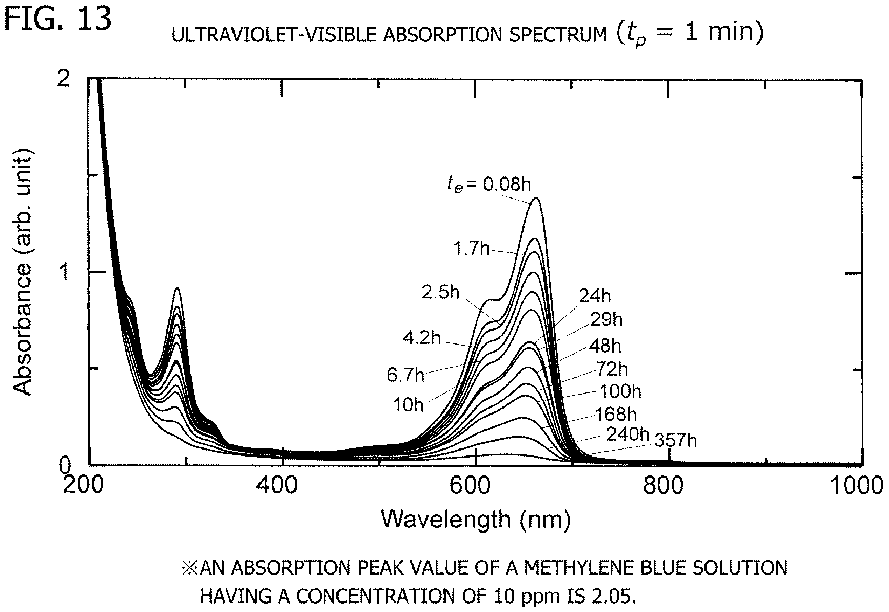

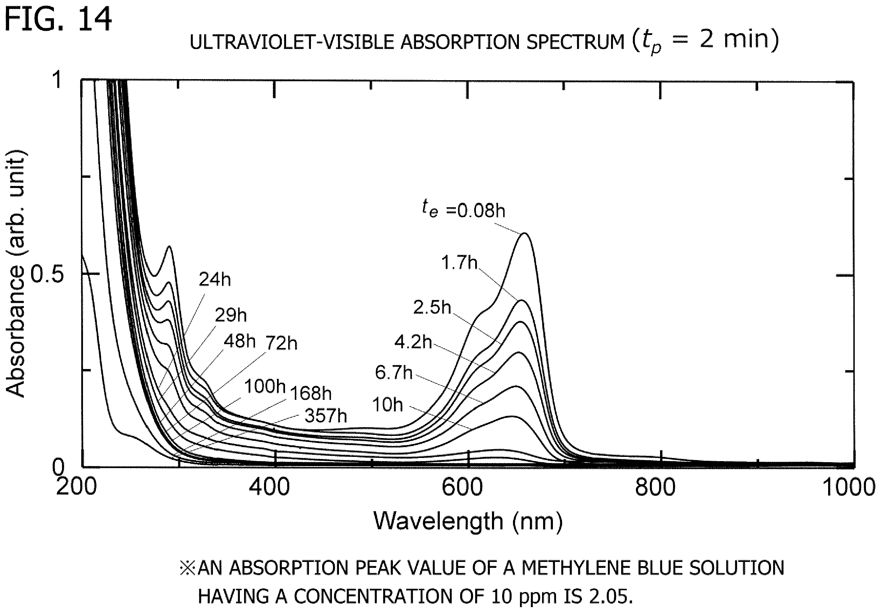

FIGS. 12 to 14 show ultraviolet-visible absorption spectra (relations between a wavelength and an absorptance for each elapsed time) of MB solutions processed under the processing conditions (tₚ = 0.5 min, 1 min, and 2 min) for a sterilizing liquid producing device shown in Table 5, respectively.

The following was revealed from the test for an organic matter decomposition effect shown in FIGS. 12 to 14.
- Reactive oxygen species produced during plasma processing are consumed during the plasma processing by decomposing contaminants, and the amount of reactive oxygen species remaining after the plasma processing is reduced, so that a sterilizing effect is lowered as a whole.
- In the case where CBP processing time is short (tₚ = 0.5 min or 1 min) and the produced amounts of reactive oxygen species (see paragraph [0018]) and nanoparticle catalyst are small, the reactive oxygen species are consumed in an initial stage, making it difficult to obtain the organic matter decomposition effect after stop. This suggests that the organic matter decomposition effect (the sterilizing effect) is greatly affected by residues (contaminations) such as organic matter in a sterilizing liquid producing device and is also affected by organic matter or the like remaining after production of a sterilizing liquid.
- In the case where the CBP processing time is long (tₚ = 2 min) and the produced amounts of reactive oxygen species and nanoparticle catalyst are large, the reactive oxygen species remaining after the stop provide the organic matter decomposition effect even when the reactive oxygen species are consumed in the initial stage.

### [Sterilizing effect test (2)]

Next, a sterilizing effect test (2) was conducted using an aqueous solution of organic matter (methylene blue) in place of microorganisms in the following procedure.

This sterilizing effect test (2) was conducted to examine a duration of a sterilizing effect of a sterilizing liquid.

Table 6 shows a processing condition for a sterilizing liquid producing device.

**[Table 6]**

| CBP Processing Condition | | |
|---|---|---|
| Solution | Type | Ion-Exchanged Water |
| | Weight | 260 g |
| | Initial pH | 6 |
| | Initial Conductivity | 1 µS/cm |
| | Initial Water Temperature | 30 °C |
| Diffusion Device | Number of Revolutions | 7200 rpm |
| Power Supply | Voltage Peak Value | 10 kV |
| | Pulse Width | 1.0 µs |
| | Repetition Frequency | 200 kHz |
| | Polarity | Bipolar |
| Electrode | Material | W |
| | Diameter | *ϕ* 2.0 mm |
| | Gap Length | 1.0 mm |
| Processing Time *tₚ* | | 1 to 30 min |

In the following test, Table 6 (tₚ = 5 min) was set as a standard condition, and a method including the same pre-processing as that in the sterilizing effect test (1) was adopted.

Next, an organic matter decomposition effect test was conducted in the following procedure.

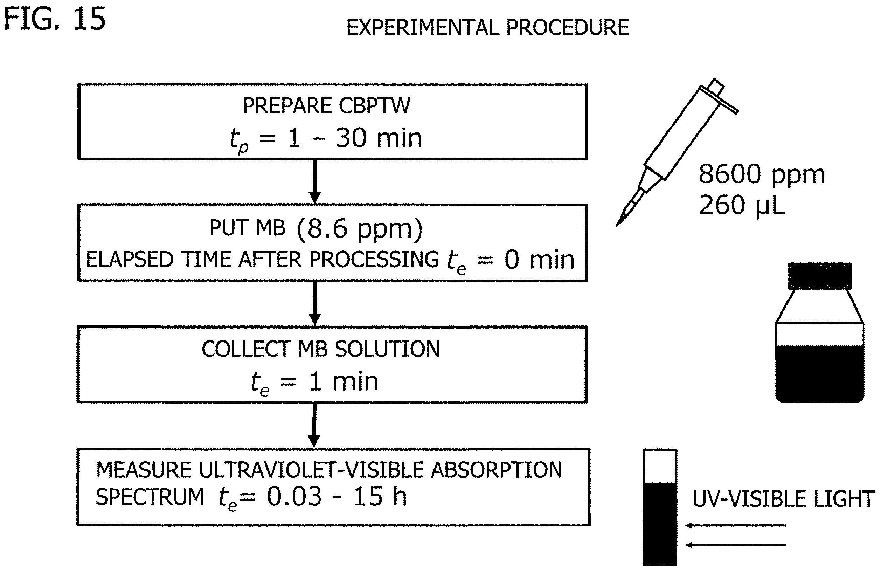

Immediately after processing under the processing condition for a sterilizing liquid producing device shown in Table 6, 260 µL of an MB solution having a concentration of 8600 ppm was put into the device, as shown in FIG. 15. Change of a peak intensity at a wavelength of 664 nm in an ultraviolet-visible absorption spectrum of the MB solution after the MB solution was put with time (tₑ = 0.03 to 15 h (hours)) was measured using an ultraviolet-visible spectrophotometer (V-730BIO, JASCO Corporation), and an MB decomposition rate for each elapsed time after production of a sterilizing liquid was calculated.

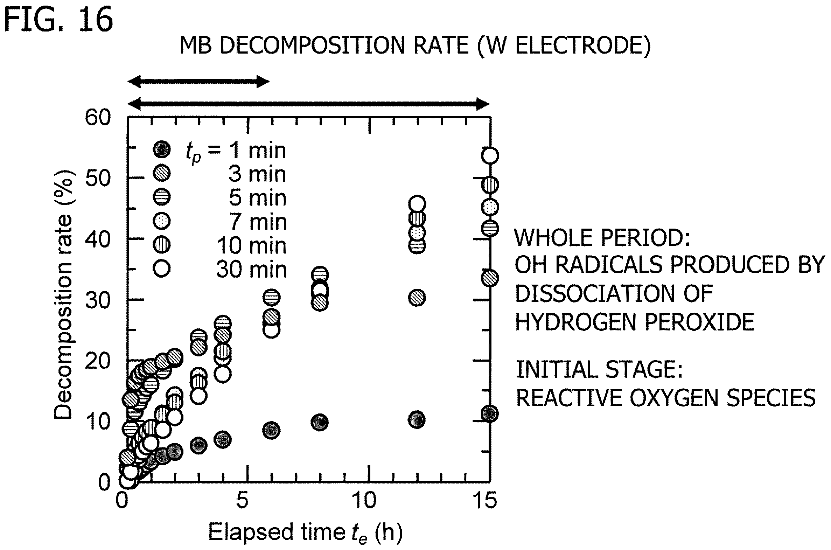

FIG. 16 shows a relation between elapsed time after production of a sterilizing liquid and an MB decomposition rate for each processing condition (tₚ = 1 min to 30 min) for a sterilizing liquid producing device.

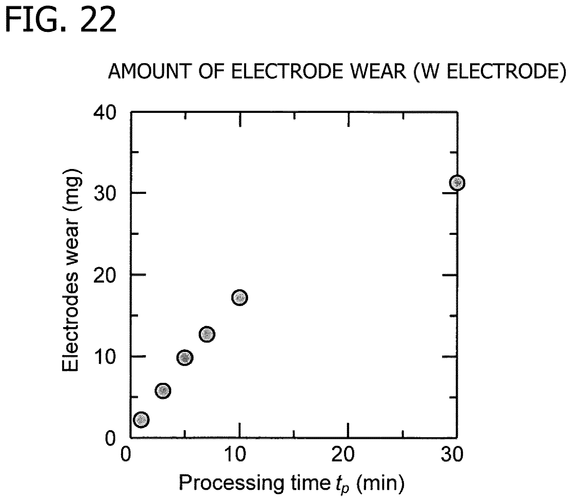

From FIG. 16, it is found that an MB decomposition effect at tₑ = 15 h is higher as processing time is longer. However, in a region of tₑ < 2 h, the best MB decomposition rate is obtained when tₚ = 3 min. This suggests that the dominant factor of the MB decomposition effect is different before and after tₑ = 2 h. Although the factors of MB decomposition will be described below, it was confirmed that a sterilizing effect of a sterilizing liquid lasts for more than a few hours (15 hours or more from the test result in FIG. 16 or dozens of hours or more from the test results in FIGS. 22 to 26 described later) due to involvement of reactive oxygen species present at the time of production of the sterilizing liquid in a region of tₑ < 2 h or involvement of the above reactive oxygen species and OH radicals produced by dissociation of hydrogen peroxide contained in the sterilizing liquid in a region of tₑ > 2 h. Here, a storage temperature of the produced sterilizing liquid is 25 °C. The sterilizing effect of the sterilizing liquid can be maintained by storing the sterilizing liquid at room temperature (normal temperature without cooling or heating). Since a reaction speed of a dissociation reaction of hydrogen peroxide described above decreases as the storage temperature is lower similarly to a normal chemical reaction, it is considered that the duration of the sterilizing effect of the sterilizing liquid can be extended by maintaining the storage temperature low.

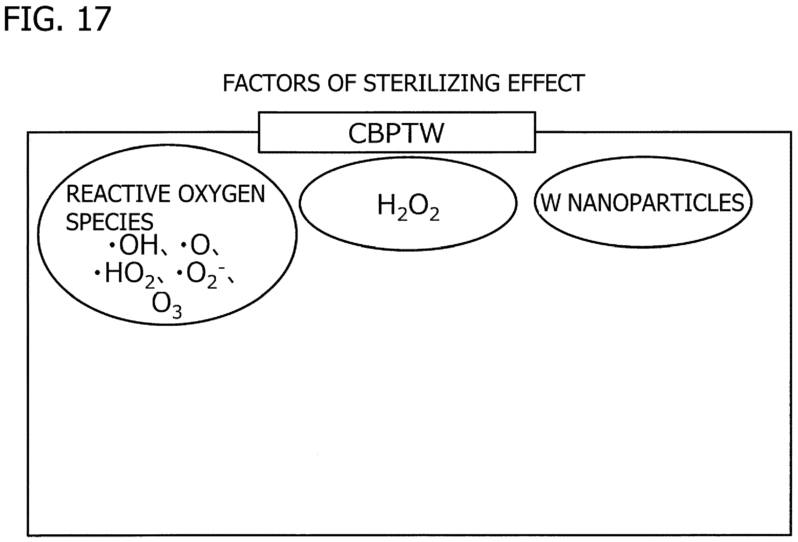

That is, as shown in FIG. 17, reactive oxygen species (·OH, ·O, ·HO₂, ·O₂⁻, O₃) (it is considered that OH radicals and O radicals are produced in plasma-processed water processed under a processing condition for a sterilizing liquid producing device as described with reference to FIGS. 6 and 7, and those radicals react with water to produce reactive oxygen species), hydrogen peroxide (H₂O₂), and tungsten (W) nanoparticles (nanoparticle catalyst) are present in a sterilizing liquid (CBPTW) that is CBP-processed water.

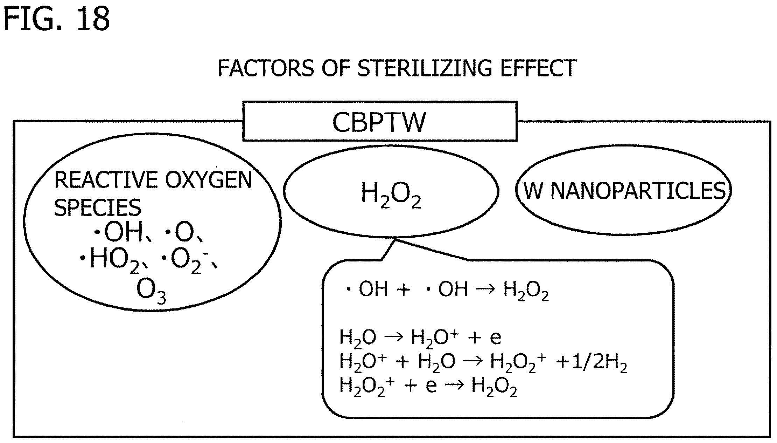

Here, it is considered that hydrogen peroxide (H₂O₂) is produced by performing CBP processing for water, as shown in FIG. 18.

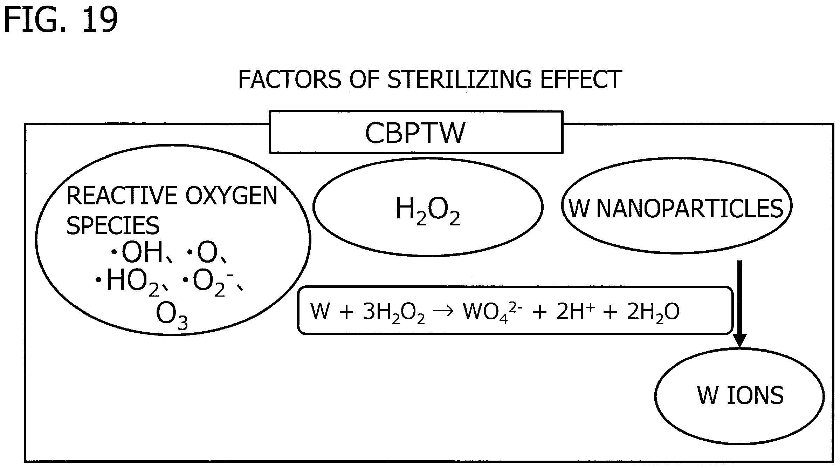

Further, a part of tungsten (W) nanoparticles (a nanoparticle catalyst) is present as tungsten (W) ions under the presence of hydrogen peroxide (H₂O₂), as shown in FIG. 19.

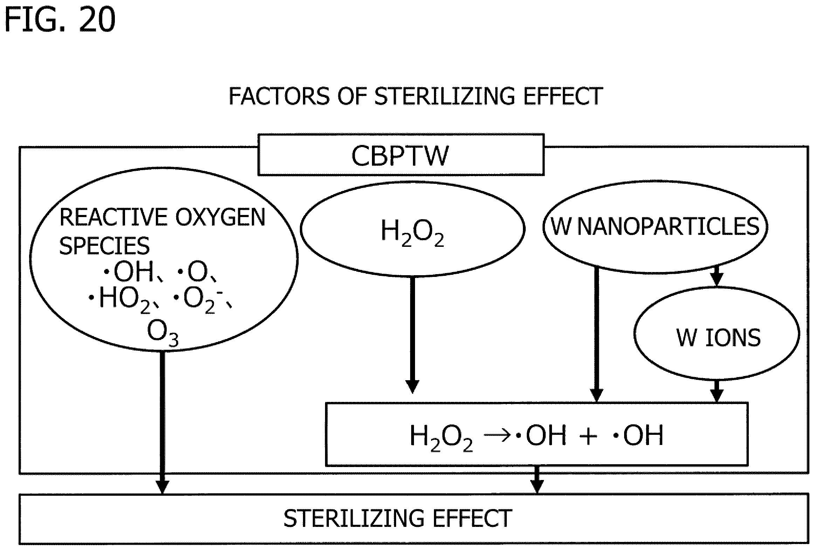

Furthermore, by an action of the tungsten (W) nanoparticles (the nanoparticle catalyst) and the tungsten (W) ions, hydrogen peroxide (H₂O₂) dissociates to produce hydroxyl radicals (·OH) that are OH radicals, as shown in FIG. 20.

Consequently, a sterilizing effect of a sterilizing liquid lasts a long time (specifically, more than a few hours (16 hours or more from the test result in FIG. 16 or dozens of hours or more from the test results in FIGS. 22 to 26 described later)) due to involvement of reactive oxygen species present at the time of production of the sterilizing liquid in an initial stage or involvement of these reactive oxygen species and OH radicals produced by dissociation of hydrogen peroxide contained in the sterilizing liquid after a certain period of time has elapsed after production of the sterilizing liquid.

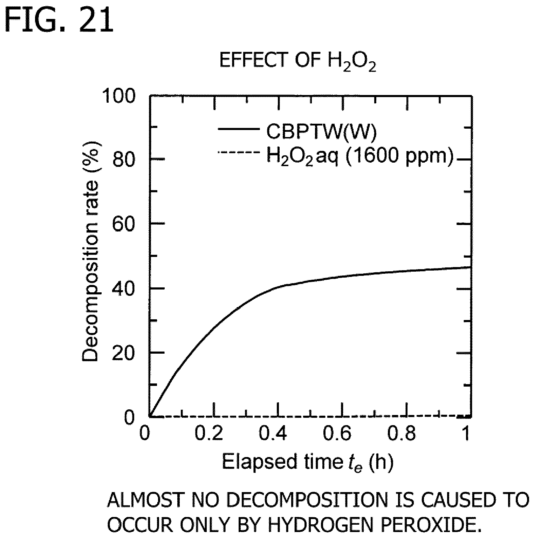

Here, it is apparent from the result of a comparative test for a sterilizing liquid that is CBP-processed water (CBPTW) and a hydrogen peroxide solution, shown in FIG. 21, that the tungsten (W) nanoparticles (a nanoparticle catalyst) contribute to decomposition of organic matter (methylene blue) (sterilization) by causing hydrogen peroxide contained in the sterilizing liquid to dissociate and produce hydroxyl radicals (-OH) (this effect cannot be obtained by hydrogen peroxide alone) .

A relation between a processing condition for a sterilizing liquid producing device in the sterilizing effect test (2), the amount of electrode wear, and the water quality (with no MB solution added) is described here, with reference to FIGS. 22 to 26.

FIG. 22 shows a relation between a processing condition for a sterilizing liquid producing device (tₚ = 1 min to 30 min) and the amount of wear of a tungsten (W) electrode.

As shown in FIG. 22, the tungsten (W) electrode is worn about 1.8 mg at tₚ = 1 min. The amount of wear then increases linearly in a range of tₚ = 1 to 10 min, and reaches about 17.3 mg at tₚ = 10 min. Thereafter, the slope of the increase becomes gentle, and the amount of wear is about 32.5 mg at tₚ = 30 min. It is considered that the slope becomes gentler with increase in the processing time because a conductivity of a solution increases and a plasma generation rate decreases. The worn electrode is present as tungsten (W) nanoparticles or tungsten (W) ions in a sterilizing liquid that is CBP-processed water (CBPTW). From this, the concentration of a nanoparticle catalyst contained in the sterilizing liquid is 130 ppm at tₚ = 30 min. Further, considering that the nanoparticle catalyst contributes to dissociation of hydrogen peroxide (H₂O₂) (production of hydroxyl radicals (·OH)), it can be said that when the sterilizing liquid is used a few hours after production of the sterilizing liquid, the concentration of the nanoparticle catalyst is preferably 70 ppm or more (tₚ = 10 min or more) and more preferably 130 ppm or more (tₚ = 30 min or more).

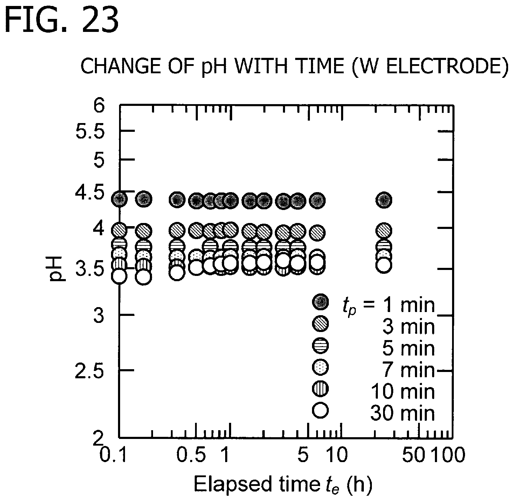

FIG. 23 shows a relation between elapsed time after production of a sterilizing liquid and a pH value for each processing condition (tₚ = 1 min to 30 min) for a sterilizing liquid producing device.

When tₚ = 1 min, the pH value is 4.4, and remains substantially constant irrespective of increase in the elapsed time. It is found that the pH value decreases with increase in the plasma processing time, but is substantially constant at any elapsed time after processing except for the case of tₚ = 30 min. In the case of tₚ = 30 min, the pH value is 3.3 at tₑ = 0.03 h, then increases until tₑ = 1 h, and thereafter is substantially constant to be 3.6. It is considered that the cause of decrease in the pH value with increase in the processing time is that tungsten reacts with water or hydrogen peroxide to produce H⁺.

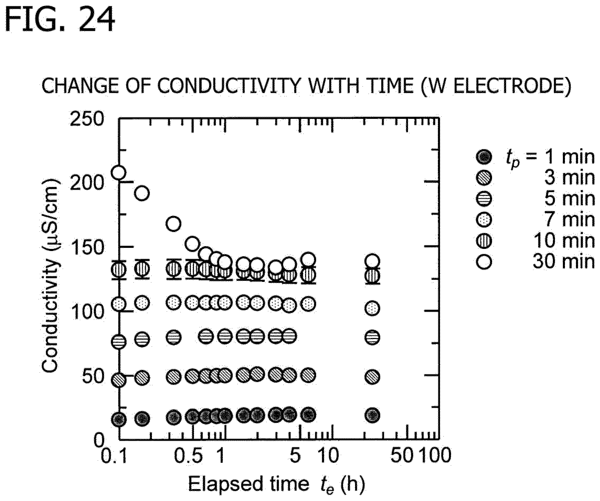

FIG. 24 shows a relation between elapsed time after production of a sterilizing liquid and a conductivity for each processing condition (tₚ = 1 min to 30 min) for a sterilizing liquid producing device.

When tₚ = 1 min, the conductivity is about 16 µS/cm at tₑ = 0.1 h, increases until tₑ = 1.5h, and tends to be saturated after reaching about 18.5 µS/cm. Thereafter, the conductivity decreases little by little and is about 18.3 µS/cm at tₑ = 24 h. When tₚ = 5 min or less, change of the conductivity with time has the same tendency as that when tₚ = 1 min. However, when tₚ = 7 min, the conductivity decreases as the elapsed time after processing increases. When tₚ = 30 min, the decrease is particularly remarkable. The conductivity rapidly decreases until tₑ = 1 h, that is, the conductivity that is 200 µS/cm at tₑ = 0.1 h decreases to 140 µS/cm at tₑ = 1 h. Thereafter, the conductivity tends to be saturated and is 138 µS/cm at tₑ = 24 h. The cause of the decrease in conductivity is considered to be that W dissolves in water having a high hydrogen peroxide concentration. It is considered that W is precipitated in the CBP-processed water to cause decrease of W ions in a solution and to lower the conductivity.

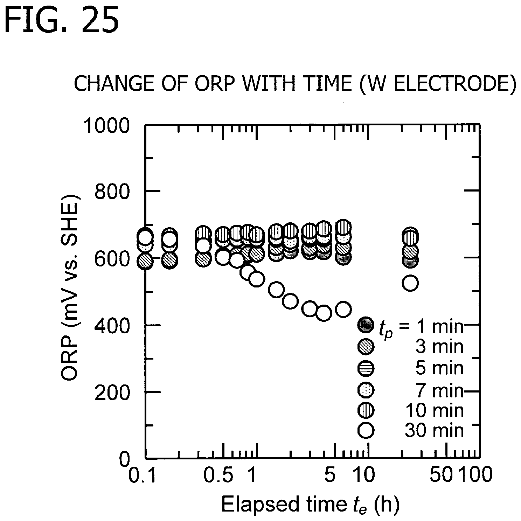

FIG. 25 shows a relation between elapsed time after production of a sterilizing liquid and an ORP (Oxidation-Reduction Potential) for each processing condition (tₚ = 1 min to 30 min) for a sterilizing liquid producing device.

When tₚ = 1 min, the ORP is about 588 mV at tₑ = 0.1 h, increases little by little with increase in elapsed time after processing, and increases to about 592 mV at tₑ = 24 h. Further, when tₚ = 7 min or less, the ORP increases little by little with increase in the elapsed time after processing, as with the tendency when tₚ = 1 min. However, when tₚ = 10 min or more, the ORP decreases with increase in the elapsed time after processing. It is considered that hydrogen peroxide is involved in this tendency. Hydrogen peroxide is decomposed into OH radicals by being added to metal powder. OH radicals are an oxidant and are involved in the increase in the ORP. From this, it is considered that the ORP value is increased by decomposition of hydrogen peroxide. Therefore, it is considered that the ORP decreased due to decrease in hydrogen peroxide and decrease in produced OH radicals.

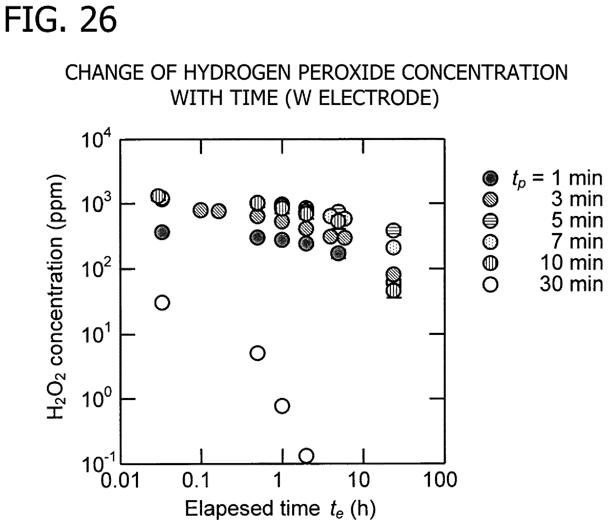

FIG. 26 shows a relation between elapsed time after production of a sterilizing liquid and a hydrogen peroxide concentration for each processing condition (tₚ = 1 min to 30 min) for a sterilizing liquid producing device.

Hydrogen peroxide is produced by a reaction of water during plasma production. When tₚ = 1 min, the hydrogen peroxide concentration is about 360 ppm at tₑ = 0.03 h, decreases exponentially with increase in elapsed time after processing, and is about 60 ppm at tₑ = 24 h. Although the hydrogen peroxide concentration decreased exponentially with regard to all processing times, the same level of hydrogen peroxide concentration was shown at tₑ = 0.03 h (about 30 ppm when tₚ = 30 min). When tₚ = 5, 7, and 10 min, the hydrogen peroxide concentration at tₑ = 30 h became lower as the processing time was longer. It is known that hydrogen peroxide dissociates into OH radicals in an environment of metal ions. As for the hydrogen peroxide concentration, it is considered that due to the above factors, a dissociation speed increases as the processing time increases. However, considering the relation between the elapsed time after production of a sterilizing liquid and the MB decomposition rate for each processing condition (tₚ = 1 min to 30 min) for a sterilizing liquid producing device shown in FIG. 16, it was found that the CBP processing time providing the sterilizing effect is not limited to tₚ = about 2 to 10 minutes derived from the result of the sterilizing effect test (1), and the sterilizing effect is exerted also at tₚ = 30 minutes (the concentration of hydrogen peroxide in reactive oxygen species = 30 ppm (see FIG. 26) (or more).

From the experimental results shown in FIGS. 22 to 26, it is considered that hydrogen peroxide is involved in decomposition of MB. It is considered that reactive oxygen species, hydrogen peroxide, and nanoparticles and ions of a metal are present in a sterilizing liquid that is CBP-processed water (CBPTW), and OH radicals produced by dissociation of hydrogen peroxide occurring under an environment of the metal ions are a factor of making a decomposition effect last. In addition, since the amount of electrode wear increases with increase in the processing time, it is considered that the metal nanoparticles and ions in a solution increase with increase in the processing time. Therefore, it is considered that the dissociation speed of hydrogen peroxide increases with increase in the processing time. When tₑ < 2 h, it is considered that the decomposition effect is further enhanced by relatively long-lived reactive oxygen species directly produced by CBP in addition to the above. Further, since the plasma generation rate decreases with increase in the processing time, it is considered that, as the processing time increases, the reactive oxygen species produced by CBP reach a maximum value and then decrease. Therefore, it is considered that before tₑ < 2 h, the decomposition effect was the maximum at 3 min, and thereafter increased in descending order of the processing time.

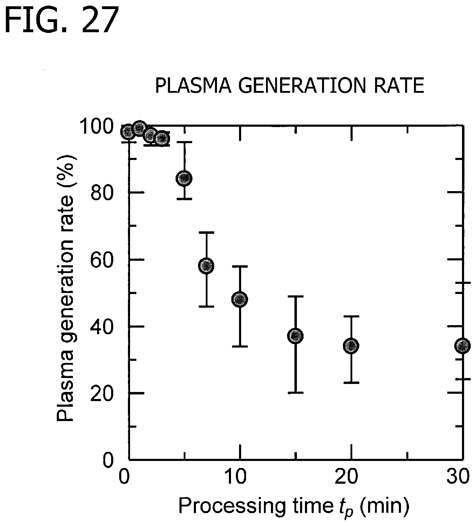

FIG. 27 shows a relation between CBP processing time tₚ (the water quality (with no MB solution added)) and a generation rate of in-liquid plasma (cavitation plasma) ((the number of pulses produced by plasma) ÷ (the number of applied pulses) × 100 [%]).

The in-liquid plasma (cavitation plasma) is produced by application of high-repetition and high-voltage pulses.

As shown in FIG. 27, the plasma generation rate tends to decrease after the CBP processing time tₚ = 3 to 5 min.

The reason for this is considered to be that a conductivity of processed water increases as CBP processing proceeds, a voltage applied across electrodes decreases in relation to the output impedance of a power supply, the probability that the applied voltage falls below a discharge inception voltage increases, and the number of times that plasma is not produced increases.

As described above, when the plasma generation rate decreases, reactive oxygen species present in an initial stage decrease. Therefore, in the case of using a sterilizing liquid within 2 hours after production of the sterilizing liquid, a preferable CBP processing time is tₚ = about 5 min.

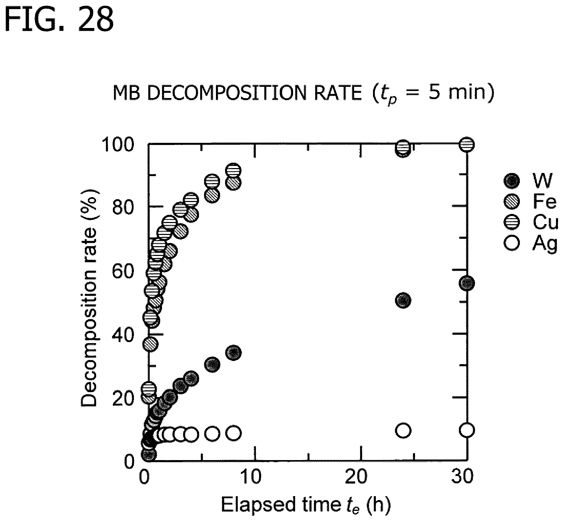

Next, a relation between an electrode material, a sterilizing effect, the amount of electrode wear, and the water quality (with no MB solution added) as for a sterilizing liquid (CBPTW) produced under the processing condition (Table 6 (tₚ = 5 min)) for a sterilizing liquid producing device in the sterilizing effect test (2), using W, Fe, Cu, and Ag as the electrode material, is described with reference to FIGS. 28 to 31.

FIG. 28 shows a relation between an electrode material and an MB decomposition rate (a sterilizing effect) (dependency on processing time).

In the case of a W electrode, the decomposition rate exponentially increases to 20% when elapsed time after processing tₑ = 0.03 to 3 h. Thereafter, the slope becomes gentle, and the decomposition rate increases to 58% at tₑ = 30 h. In the case where CBPTW was prepared with other electrodes and MB was added, the decomposition rate rapidly increased at tₑ = 0.1 to 3 h, and then showed a saturation tendency as with the W electrode. However, in the case of Cu and Fe electrodes, the decomposition rate at tₑ = 30 h increased to about 100%. In the case of the Cu and Fe electrodes, a manner of increase of the decomposition rate was also different. At tₑ = 3 h, the decomposition rate for the Cu electrode increased by about 76%, whereas the decomposition rate for the Fe electrode increased by only about 65%. These results show that an effect of decomposing MB is higher in the case where the electrode material is Cu than in the case where the electrode material is Fe. In the case where the electrode material is Ag, the decomposition rate at tₑ = 30 h is about 10%, i.e., almost no decomposition occurs. These results show that the MB decomposition effect is higher in the order of Ag <W <Fe <Cu as the electrode material.

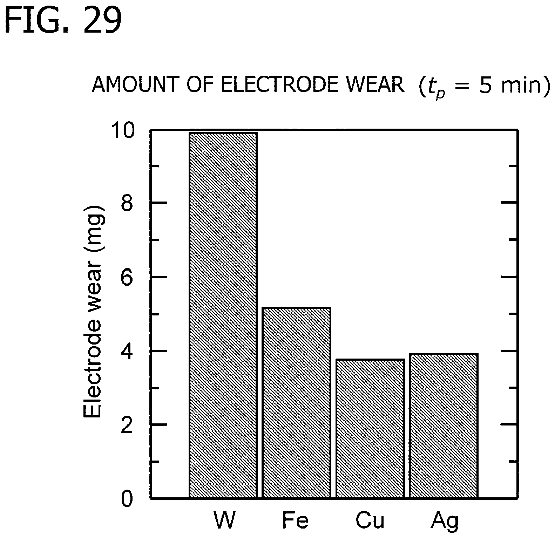

FIG. 29 shows a relation between an electrode material and the amount of electrode wear.

As shown in FIG. 29, the amounts of electrode wear are 9.8, 5.2, 3.6, and 3.8 mg when the electrodes are made of W, Fe, Cu, and Ag, respectively. It is considered that the worn electrode is present as nanoparticles or metal ions in a solution.

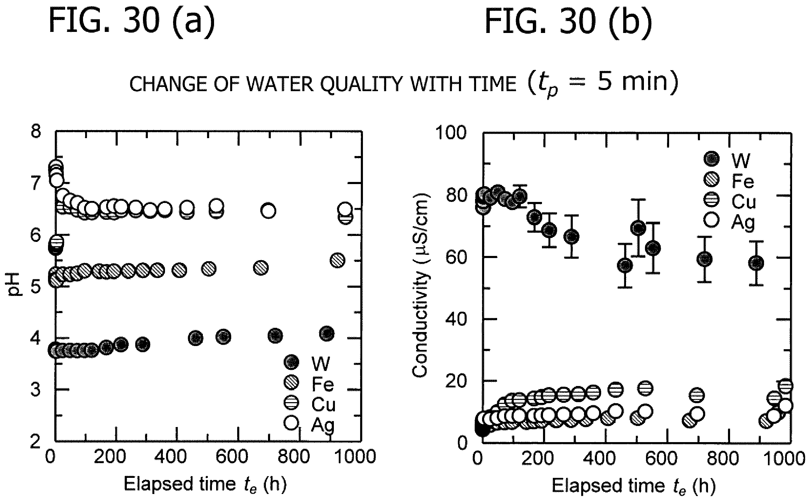

FIG. 30(a) shows a relation between an electrode material and a pH value.

As shown in FIG. 30(a), in the case of a W electrode, a pH value is about 3.8 when elapsed time after processing tₑ = 0.1 h, increases little by little with increase in the elapsed time, and increases to about 4.1 at tₑ = 900 h. For Fe, Cu and Ag electrodes, the pH values are about 5.2, 5.8, 7.2 at tₑ = 0.1 h, respectively. In addition, in the case of the Fe electrode, the pH value increases gradually as the elapsed time increases, and is about 5.5 at tₑ = 970 h. Meanwhile, in the case of the Cu and Ag electrodes, the pH values change to 6.5 at tₑ = 24 h, then tend to be saturated, and are about 6.5 at tₑ = 970h.

FIG. 30(b) shows a relation between an electrode material and a conductivity.

As shown in FIG. 30 (b), in the case of a W electrode, the conductivity is about 78 µS/cm when elapsed time after processing tₑ = 0.1 h, increases to about 80 µS/cm at tₑ = 1.5 h, then tends to be saturated until tₑ = 100 h, tends to decrease until tₑ = 450 h, and tends to be saturated after decreasing to 60 µS/cm. In the case of Fe, Cu and Ag electrodes, the conductivities were lower than that of the W electrode and were only about 8 µS/cm. The conductivities of the Fe, Cu and Ag electrodes increased gradually with increase in the elapsed time, and were about 9, 15, and 11 µS/cm at tₑ = 950 h, respectively. It was found from these results that change of the conductivity with time varies depending on the electrode material.

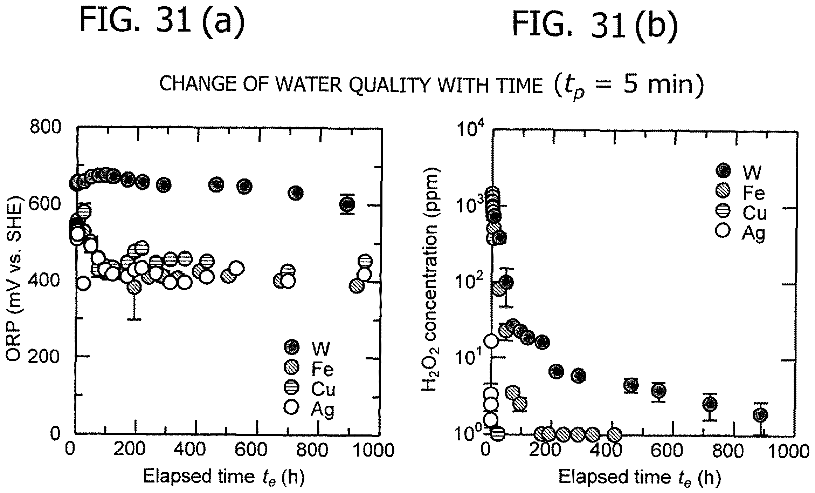

FIG. 31(a) shows a relation between an electrode material and an ORP.

As shown in FIG. 31 (a), in the case of a W electrode, the ORP is about 650 mV at tₑ = 0.1 h, increases gradually until tₑ = 100 h and reaches 680 mV, then decreases to about 600 mV at tₑ = 950 h. In the case of Fe, Cu, and Ag electrodes, the ORP are 340, 340, and 320 mV at tₑ = 0.1 h, respectively, decrease gradually with increase in the elapsed time, become 200, 230, and 210 mV at tₑ = 100 h, respectively, and then tend to be saturated. The cause of decrease in the ORP is considered to be that, since hydrogen peroxide is decomposed into OH radicals by dissociation, the ORP decreases because hydrogen peroxide disappears in CBPTW and OH radicals are no longer produced.

FIG. 32(b) shows a relation between an electrode material and a hydrogen peroxide concentration.

As shown in FIG. 32 (b), in the case of a W electrode, the hydrogen peroxide concentration is about 1200 ppm at tₑ = 0.03 and exponentially decreases until tₑ = 5 h. Thereafter, the slope becomes gentle, and the hydrogen peroxide concentration is 2 ppm at tₑ = 880 h. At tₑ = 0.03h, the hydrogen peroxide concentrations for Fe and Cu electrodes are about 1200 ppm, similar to the W electrode. However, the hydrogen peroxide concentration is out of a measurement range at tₑ = 400 h for the Fe electrode or at tₑ = 48 h for the Cu electrode. From these results, it is found that the decrease amount of the hydrogen peroxide concentration varies depending on the electrode material. Further, for an Ag electrode, the hydrogen peroxide concentration is 10 ppm at tₑ = 0.03h, and is out of a measurement range at tₑ = 4 h. Therefore, it is found that the hydrogen peroxide concentration decreases more largely as compared with the other electrodes. From these results, it is considered that a decomposition speed of the hydrogen peroxide concentration becomes larger in the order of W <Fe <Cu <Ag as the electrode material. It is also considered that, for the Ag electrodes, although hydrogen peroxide is produced in a similar manner to those in the other electrodes, the decomposition speed is large, and hydrogen peroxide is almost decomposed at tₑ = 0.03 h.

A sterilizing liquid and a method of producing the same according to the present invention have been described by way of an embodiment in the above description. However, the present invention is not limited thereto, and the configuration can be changed as appropriate without departing from the gist of the present invention.

### INDUSTRIAL APPLICABILITY

A sterilizing liquid and a method of producing the same according to the present invention are safe and secure, have no restrictions on application methods and usage, and can be widely used for sterilization because a raw material of the sterilizing material of the present invention is water, and reactive oxygen species as a component exerting a sterilizing action return to water finally. Accordingly, the sterilizing liquid and the method of producing the same according to the present invention can be widely used for sterilization (for example, for killing pathogens in farms by spraying the sterilizing liquid, for killing pathogens in plant factories, as a post-harvest sterilizing liquid for putrefactive bacteria, and for prevention of virus infection in dairy farming) in fields of agriculture, food, hygiene, medical treatment, and the like.

### DESCRIPTION OF REFERENCE NUMERALS

1 tank
2 stirrer
21 casing
22 rotor
23 motor
3 plasma generation mechanism
31 electrode
32 pulse power supply
4 conduit line

## Claims

1. A sterilizing liquid comprising water containing reactive oxygen species and a nanoparticle catalyst in a stationary state.

2. The sterilizing liquid of claim 1, wherein a concentration of hydrogen peroxide among the reactive oxygen species immediately after production of the sterilizing liquid is 30 to 2000 ppm, and a concentration of the nanoparticle catalyst is 16 ppm or more.

3. The sterilizing liquid of claim 1 or 2, wherein a sterilizing effect of the sterilizing liquid lasts for several hours or more.

4. A method of producing a sterilizing liquid, wherein the sterilizing liquid includes water containing reactive oxygen species and a nanoparticle catalyst in a stationary state, the method comprising: causing cavitation in water having a conductivity of 2000 µS/cm or less and COD of 2000 ppm or less while causing the water to flow, and generating plasma by a plasma generation mechanism in which a pulse voltage is applied across electrodes in the water including air bubbles mainly containing water vapor generated by the cavitation.
